# EUROPÄISCHE PATENTSCHRIFT

(11) **EP 2 200 575 B1**
(45) Veröffentlichungstag und Bekanntmachung des Hinweises auf die Patenterteilung: **03.09.2014**
(21) Anmeldenummer: 08804393.0
(22) Anmeldetag: 18.09.2008
(51) Int. Cl.: A61K 8/38, A61K 8/49, A61Q 5/04

(54) **KOSMETISCHES MITTEL ENTHALTEND PURIN UND/ODER EIN PURINDERIVAT UND WASSERSTOFFPEROXID**
COSMETIC AGENT CONTAINING PURINE AND/OR A PURINE DERIVATIVE AND HYDROGEN PEROXIDE
AGENT COSMÉTIQUE CONTENANT DE LA PURINE ET/OU UN DÉRIVÉ DE PURINE, ET DU PEROXYDE D'HYDROGÈNE

(30) Priorität: 22.10.2007 DE 102007050764; 12.12.2007 DE 102007060323; 13.12.2007 DE 102007060534
(43) Veröffentlichungstag der Anmeldung: 30.06.2010
(73) Patentinhaber: Henkel AG & Co. KGaA, 40589 Düsseldorf (DE)
(72) Erfinder: GASSENMEIER, Thomas, 32756 Detmold (DE); EMMERLING, Winfried, 25436 Tornesch (DE); HEYER, Michael, 40699 Erkrath (DE); AKRAM, Mustafa, 22455 Hamburg (DE)
(86) Internationale Anmeldenummer: PCT/EP2008/062455
(87) Internationale Veröffentlichungsnummer: WO 2009/053180

(56) Entgegenhaltungen:
- EP-A- 0 083 095
- WO-A-2007/076993

## Beschreibung

Die vorliegende Erfindung betrifft kosmetische Mittel enthaltend Purin und/oder Purinderivat(e) und Wasserstoffperoxid, die Verwendung dieser Mittel im Rahmen einer dauerhaften Verformung keratinischer Fasern, sowie ein Verfahren zur dauerhaften Verformung keratinischer Fasern unter Verwendung dieser Mittel.

Als keratinhaltige Fasern können prinzipiell alle tierischen Haare, z.B. Wolle, Rosshaar, Angorahaar, Pelze, Federn und daraus gefertigte Produkte oder Textilien eingesetzt werden. Vorzugsweise handelt es sich bei den keratinhaltigen Fasern jedoch um menschliche Haare und daraus gefertigte Perücken.

Eine dauerhafte Verformung keratinhaltiger Fasern wird üblicherweise derart durchgeführt, dass man die Faser mechanisch verformt und die Verformung durch geeignete Hilfsmittel festlegt. Vor und/oder nach dieser Verformung behandelt man die Fasern mit einer keratinreduzierenden Zubereitung. Nach einem Spülvorgang wird die Faser dann in dem sogenannten Fixierschritt mit einer Oxidationsmittelzubereitung behandelt, gespült und nach oder während des Fixierschritts von den Verformungshilfsmitteln (Wicklern, Papilloten) befreit. Wenn als keratinreduzierende Komponente ein Merkaptan, z.B. Ammoniumthioglykolat, verwendet wird, spaltet dieses einen Teil der Disulfid-Brücken des Keratin-Moleküls zu -SH-Gruppen, so dass es zu einer Erweichung der Keratinfaser kommt. Bei der späteren oxidativen Fixierung werden erneut Disulfid-Brücken im Haarkeratin geknüpft, so dass das Keratingefüge in der vorgegebenen Verformung fixiert wird. Alternativ ist es bekannt, zur Haarverformung anstelle der Merkaptane Sulfit zu verwenden. Durch Hydrogensulfit-Lösungen und/oder Sulfit-Lösungen und/oder Disulfit-Lösungen werden Disulfid-Brücken des Keratins in einer Sulfitolyse nach der Gleichung

R-S-S-R + HSO₃⁽⁻⁾ → R-SH + R-S-SO₃⁽⁻⁾

gespalten und auf diese Weise eine Erweichung der Keratinfaser erreicht. Hydrogensulfit-, sulfit- bzw. disulfithaltige Reduktionsmittel weisen nicht den starken Eigengeruch der merkaptanhaltigen Mittel auf. Die Spaltung kann wie zuvor geschildert in einem Fixierschritt mit Hilfe eines Oxidationsmittels unter Bildung von neuen Disulfid-Brücken wieder rückgängig gemacht werden.

Die permanente Glättung keratinhaltiger Fasern wird analog durch den Einsatz von keratinreduzierenden und -oxidierenden Zusammensetzungen erzielt. In einem entsprechenden Verfahren wird das krause Haar entweder auf Wickler mit einem großen Durchmesser von üblicherweise mehr als 15 mm gewickelt oder das Haar unter Einwirkung der keratinreduzierenden Zusammensetzung glattgekämmt. Anstelle des Wicklers ist es auch möglich, die Faser auf ein Glättungsboard glattzulegen. Glättungsboarde sind üblicherweise rechteckige Tafeln z.B. aus Kunststoff.

Die permanente Verformung keratinhaltiger Fasern mit den heute verfügbaren Mitteln stellt nach wie vor eine starke Beanspruchung der behandelten Fasern dar. Insbesondere der übliche Einsatz von Fixiermitteln, die eine hohe Konzentration Wasserstoffperoxid enthalten, belastet die Fasern und kann diese merklich schädigen. Es muss bei der permanenten Verformung daher darauf geachtet werden, dass die Fasern möglichst schonend behandelt werden, dass aber gleichzeitig ein gleichmäßiges und beständiges Umformungsergebnis erhalten wird.

Um die Schädigung der keratinhaltigen Fasern durch eine permanente Verformung möglichst gering zu halten, wurde bereits mehrfach vorgeschlagen, der keratinreduzierenden Zusammensetzung und/oder dem Fixiermittel eine konditionierend wirkende Verbindung zuzusetzen. Der Einsatz einer Vielzahl solcher Verbindungen, sowie von Mischungen verschiedener konditionierend wirkender Verbindungen ist bekannt. Der schädigende Einfluß von Wasserstoffperoxid auf die Faser kann so abgemildert werden. Alternativ kommen separate Pflegezubereitungen zum Einsatz, die in Form spezieller Vor-, Zwischen- oder Nachbehandlungsmittel Verwendung finden. Die Zugabe konditionierender Verbindungen zu den Wirkzusammensetzungen, d.h. dem Well- bzw. Verformungsmittel und/oder dem Fixiermittel führt oftmals zu einer Verschlechterung der Verformungsleistung. Bei Verwendung spezieller Vor-, Zwischen- oder Nachbehandlungsmittel kann dieses Problem vermieden werden. Durch Einsatz eines solchen zusätzlichen Mittels wird der Verformungsvorgang jedoch deutlich aufwändiger.

Es wurde daher bereits verschiedentlich der Ersatz von Wasserstoffperoxid durch andere Oxidationssysteme vorgeschlagen.

So offenbart etwa WO 2007/076993 A1 ein Verfahren zur Ausbildung von Disulfidbrücken bei SH-Gruppen tragenden Substanzen, bei dem eine heterozyklische, wenigstens ein Stickstoffatom aufweisende Verbindung, insbesondere Koffein oder eine koffeinähnliche Substanz zur Katalyse der Reaktion eingesetzt wird. In Gegenwart dieser heterozyklischen Verbindungen kommt es zu einer schonenden Oxidation allein durch die Gegenwart von Luftsauerstoff. Das Verfahren eignet sich insbesondere zur Schließung der Disulfidbrücken im Rahmen einer Dauerwellbehandlung. In diesem Fall wird es als besonders vorteilhaft angesehen, zur Förderung der Disulfidbrückenbildung ein Oxidationsmittel zuzugeben. Dadurch kann die Reaktionsgeschwindigkeit deutlich beschleunigt werden. Das einzige explizit genannte geeignete Oxidationsmittel ist oxidiertes Glutathion.

Es hat sich nun jedoch gezeigt, dass der alleinige Einsatz der in WO 2007/076993 A1 genannten heterozyklischen, wenigstens ein Stickstoffatom aufweisenden Verbindung in Mitteln zur Durchführung der oxidativen Stufe eines Verfahrens zur dauerhaften Verformung keratinischer Fasern nicht zu zufrieden stellenden Verformungsergebnissen führt. Wellwirkung bzw. die Haltbarkeit der Verformung, insbesondere gegenüber Feuchtigkeit, können nicht überzeugen. Durch Zugabe von oxidiertem Glutathion kann die Reaktionsgeschwindigkeit beschleunigt werden. Wellwirkung und die Haltbarkeit der Verformung sind jedoch ebenfalls nicht zufrieden stellend. Zudem ist auch bei Einsatz von Fixiermitteln, die Koffein oder eine koffeinähnliche Substanz enthalten und frei von Wasserstoffperoxid sind, noch eine beträchtliche Haarschädigung zu verzeichnen.

Aufgabe der vorliegenden Erfindung war es daher, kosmetische Mittel zur Verfügung zu stellen, die sich zur Durchführung der oxidativen Stufe einer permanenten Verformung eignen, wobei die Fasern einerseits möglichst schonend behandelt werden, andererseits aber auch ein gleichmäßiges und dauerhaftes Umformungsergebnis erhalten wird.

Es wurde nunmehr überraschenderweise gefunden, dass dies durch kosmetische Mittel erreicht werden kann, die Wasserstoffperoxid in einer für Fixiermittel üblichen Menge in Kombination mit Purin und/oder Purinderivaten enthalten.

Ein erster Gegenstand der vorliegenden Erfindung ist daher ein kosmetisches Mittel, enthaltend in einem kosmetisch akzeptablen Träger, jeweils bezogen aus das gesamte Mittel
a) 0,03 bis 10 Gew.-% Purin und/oder Purinderivat(e) und
b) Wasserstoffperoxid in einer Menge von 0,001 bis höchstens 5 Gew.-%.

Es hat sich überraschenderweise gezeigt, dass die Zugabe von Purin und/oder Purinderivaten zu herkömmlichen Fixiermitteln auf Wasserstoffperoxidbasis eine deutliche Verbesserung der Wellleistung und Beständigkeit des Verformungsergebnisses mit sich bringt. Zudem verläuft die Behandlung der Fasern im Vergleich zum Einsatz klassischer Fixiermittel ohne Purin und/oder Purinderivat deutlich schonender.

Als ersten zwingenden Bestandteil enthalten die erfindungsgemäßen kosmetischen Mittel Purin und/oder mindestens ein Purinderivat bezogen auf das gesamte Mittel in einer Menge von 0,03 bis 10 Gew.-%.

Dabei werden Purin und/oder Purinderivat(e), jeweils bezogen auf das gesamte Mittel, vorzugsweise in Mengen von 0,3 bis 10 Gew.-%, bevorzugt von 0,4 bis 5 Gew.-%, besonders bevorzugt 0,5 bis 2,5 Gew.-% eingesetzt.

Vorzugsweise enthalten die Mittel Purin und/oder Purinderivat(e) der Formel (I) 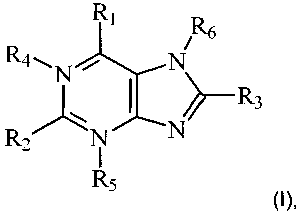 wobei die Reste R¹, R² und R³ unabhängig voneinander ausgewählt sind aus -H, - OH, -NH₂, - SH und die Reste R⁴, R⁵ und R⁶ unabhängig voneinander ausgewählt sind aus -H, -CH₃ und - CH₂-CH₃.

Purin (7H-Imidazo[4,5-d]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden. Purine und Purinderivate werden oft aus Naturstoffen isoliert, sind aber auch auf vielen Wegen synthetisch zugänglich.

Geeignete Verbindungen der Formel (I) sind insbesondere:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Koffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H)

Purinderivate der Formel (I), wobei R¹ und R² für - OH und R³ für -H stehen, werden auch als Xanthin und/oder Xanthinderivate bezeichnet. Die erfindungsgemäßen Mittel enthalten als Purinderivate vorzugsweise Xanthin und/oder Xanthinderivate.

Weiterhin sind solche Verbindungen der Formel (I) bevorzugt, in denen R⁴, R⁵ und R⁶ unabhängig voneinander ausgewählt sind aus -H und -CH₃.

Besonders bevorzugte Verbindungen der Formel (I) sind:
- Xanthin (R¹ = R² = OH, R³ = R⁴ = R⁵ = R⁶ = H)
- Koffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H)

Ganz besonders bevorzugt enthalten die erfindungsgemäßen Mittel als Purin und/oder Purinderivat Koffein.

Natürlich können die erfindungsgemäßen Mittel auch mehrere Komponenten ausgewählt aus Purin und Purinderivaten enthalten, wobei wiederum Kombinationen bevorzugt sind, die Koffein enthalten.

Als zweiten zwingenden Bestandteil enthalten die erfindungsgemäßen Mittel Wasserstoffperoxid in einer Menge von 0.001 bis 5 Gew.-% bezogen auf das gesamte Mittel.

Wasserstoffperoxid wird dabei vorzugsweise als wässrige Lösung eingesetzt. Zur Stabilisierung dieser Lösungen können übliche Stabilisatoren zugesetzt werden.

Im Rahmen einer ersten bevorzugten Ausführungsform wurde gefunden, dass eine Kombination von Purin und/oder Purinderivaten mit 0,001 bis 0,5 Gew.-% Wasserstoffperoxid (bezogen auf das gesamte Mittel) in deutlich verstärktem Masse eine Verbesserung der Wellleistung und Beständigkeit des Verformungsergebnisses mit sich bringt. Die Behandlung der Fasern verläuft sowohl im Vergleich zum Einsatz klassischer Fixiermittel mit einem Gehalt an Wasserstoffperoxid von etwa 2,5 Gew.-%, als auch im Vergleich zum Einsatz von Fixiermitteln, die Purin und/oder Purinderivat(e) als einziges Oxidationsmittel enthalten, deutlich schonender.

Die erfindungsgemäßen Mittel dieser Ausführungsform enthalten Purin und/oder Purindervat(e) bevorzugt in einer Menge von 0,3 bis 10 Gew.-%, besonders bevorzugt von 0,4 bis 5 Gew.-%, ganz besonders bevorzugt 0,5 bis 2,5 Gew.-%, jeweils bezogen auf das gesamte Mittel.

Die erfindungsgemäßen Mittel dieser Ausführungsform enthalten Wasserstoffperoxid bezogen auf das gesamte Mittel vorzugsweise in Mengen von 0,1 bis 0,4 Gew.-%, besonders bevorzugt in Mengen von 0,1 bis 0,35 Gew.-%, ganz besonders bevorzugt in Mengen von 0,1 bis 0,3 Gew.-%.

Die erfindungsgemäßen Mittel dieser Ausführungsform enthalten Purin und/oder Purindervat(e) bevorzugt in einer Menge von 0,3 bis 10 Gew.-%, besonders bevorzugt von 0,4 bis 5 Gew.-%, ganz besonders bevorzugt 0,5 bis 2,5 Gew.-%, jeweils bezogen auf das gesamte Mittel.

Selbst bei sehr geringen Mengen Wasserstoffperoxid konnte o.g. Effekt festgestellt werden, wenn auch abgeschwächt. In einer möglichen Ausführungsform liegt der Gehalt an Wasserstoffperoxid bei 0,001 bis 0,1 Gew.-%, vorzugsweise 0,005 bis 0,075 Gew.-%, besonders bevorzugt 0,0075 bis 0,05 Gew.-%, jeweils bezogen auf das gesamte Mittel.

Die erfindungsgemäßen Mittel dieser Variante der ersten Ausführungsform enthalten Purin und/oder Purindervat(e) bevorzugt in einer Menge von 0,3 bis 10 Gew.-%, besonders bevorzugt von 0,4 bis 5 Gew.-%, ganz besonders bevorzugt 0,5 bis 2,5 Gew.-%, jeweils bezogen auf das gesamte Mittel.

Im Rahmen einer zweiten bevorzugten Ausführungsform konnte gefunden werden, dass bei Kombination von Purin und/oder Purinderivat(en) mit mehr als 0,5 Gew.-% Wasserstoffperoxid, insbesondere mit mehr als 0,5 bis 5 Gew.-% Wasserstoffperoxid (jeweils bezogen auf das gesamte Mittel) eine Reduktion der Haarschädigung durch die Kombination bewirkt wird. Dieser Effekt ist immer noch deutlich. Die Wellwirksamkeit und die Haltbarkeit der Formgebung kann durch Einsatz der erfindungsgemäßen Kombination im Rahmen dieser Ausführungsform verbessert werden. Die Steigerung des Wellergebnisses fällt jedoch im Vergleich zur ersten Ausführungsform geringer aus.

Im Rahmen der zweiten Ausführungsform enthalten die erfindungsgemäßen Mittel Wasserstoffperoxid bezogen auf das gesamte Mittel vorzugsweise in Mengen von 0,7 bis 4,0 Gew.-%, besonders bevorzugt in Mengen von 1,0 bis 3,0 Gew.-%, ganz besonders bevorzugt in Mengen von 2,0 bis 3,0 Gew.-%.

Hierbei ist es wiederum bevorzugt, die Purin und/oder Purinderivat(e), jeweils bezogen auf das gesamte Mittel, in Mengen von 0,3 bis 10 Gew.-%, besonders bevorzugt von 0,4 bis 5 Gew.-%, ganz besonders bevorzugt 0,5 bis 2,5 Gew.-% einzusetzen.

Die erfindungsgemäßen Mittel enthalten Purin und/oder Purinderivat(e) und Wasserstoffperoxid in einem kosmetisch akzeptablen Träger.

Bevorzugte kosmetisch akzeptable Träger sind wässrige, alkoholische oder wässrigalkoholische Medien. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können in der wässrig alkoholischen Basis in einem Gewichtsverhältnis von 1:10 bis 10:1 vorliegen. Wasser sowie wässrig-alkoholische Mischungen, die bis zu 50 Gew.-%, insbesondere bis zu 25 Gew.-%, Alkohol, bezogen auf das Gemisch Alkohol/Wasser, enthalten, sind erfindungsgemäß bevorzugte Träger.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Der pH-Wert dieser Zubereitungen liegt bevorzugt zwischen 2 und 6. Die Angaben zum pH-Wert beziehen sich dabei im Sinne dieser Schrift auf den pH-Wert bei 25°C, sofern nichts anderes vermerkt ist. Zur Einstellung dieses pH-Wertes kann praktisch jede für kosmetische Zwecke verwendbare Säure oder Base verwendet werden. Üblicherweise werden als Säuren Genusssäuren verwendet. Unter Genusssäuren werden solche Säuren verstanden, die im Rahmen der üblichen Nahrungsaufnahme aufgenommen werden und positive Auswirkungen auf den menschlichen Organismus haben. Genusssäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Triethanolamin sowie N,N,N',N'-Tetrakis-(2-hydroxypropyl)-ethylendiamin.

Die erfindungsgemäßen Mittel können weiterhin die Hilfs- und Zusatzstoffe enthalten, die üblicherweise den jeweiligen kosmetischen Mitteln zugesetzt werden.

Als geeignete Hilfs- und Zusatzstoffe sind insbesondere Pflegestoffe zu nennen.

Als Pflegestoff kann beispielsweise ein Silikonöl und/oder ein Silikongum eingesetzt werden. In einer besonderen Ausführungsform der Erfindung enthalten die Mittel mindestens ein Silikonöl und/oder ein Silikongum.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Silicium-organischer Verbindungen. Zunächst werden hierunter die Dimethiconole (S1) verstanden. Diese können sowohl linear als auch verzweigt als auch cyclisch oder cyclisch und verzweigt sein. Lineare Dimethiconole können durch die folgende Strukturformel (S1 - I) dargestellt werden:

(HOSiR¹₂) - O - (SiR²₂ - O -)ₓ - (Si R¹₂OH) (S1 - I)

Verzweigte Dimethiconole können durch die Strukturformel (S1 - II) dargestellt werden:

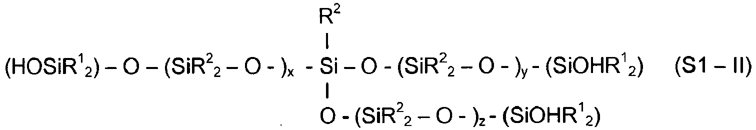

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Die Molgewichte der Dimethiconole liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Dimethicone (S2) bilden die zweite Gruppe der Silikone, welche erfindungsgemäß enthalten sein können. Diese können sowohl linear als auch verzweigt als auch cyclisch oder cyclisch und verzweigt sein. Lineare Dimethicone können durch die folgende Strukturformel (S2 - I) dargestellt werden:

(SiR¹₃) - O - (SiR¹R²-O-)ₓ-(SiR¹₃) (S2-I)

Verzweigte Dimethicone können durch die Strukturformel (S2-II) dargestellt werden:

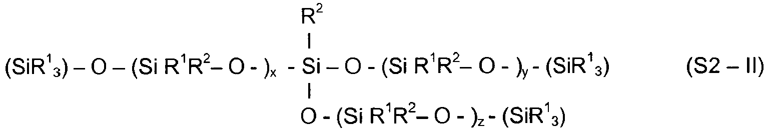

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Nicht einschränkende Bevorzugte Viskositäten liegen zwischen 1000 und 5000000 cPs, besonders bevorzugte Viskositäten liegen zwischen 10000 und 3000000 cPs. Ganz besonders bevorzugt liegt die Viskosität im Bereich zwischen 50000 und 2000000 cPs.

Dimethiconcopolyole (S3) bilden eine weitere Gruppe von Silikonen, die geeignet sind. Dimethiconcopolyole können durch die folgenden Strukturformeln dargestellt werden:

(SiR¹₃)-O-(SiR²₂-O-)ₓ-(SiR²PE-O-)_{y}-(SiR¹₃) (S3-I),

PE - (SiR¹₂)-O-(SiR²₂-O-)ₓ-(SiR¹₂)-PE (S3-II)

Verzweigte Dimethiconcopolyole können durch die Strukturformel (S3 - III) dargestellt werden: 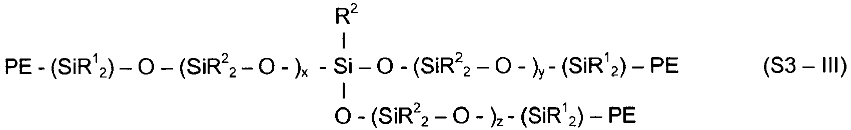 oder durch die Strukturformel (S3 - IV):

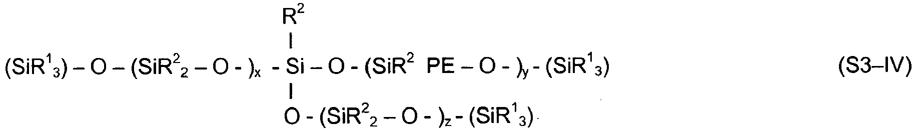

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Entsprechende Dimethiconcopolyole sind kommerziell erhältlich und werden beispielsweise von der Firma Dow Corning unter der Bezeichnung Dow Corning^{®} 5330 Fluid vertrieben.

Selbstverständlich können die Dimethiconole, Dimethicone und/oder Dimethiconcopolymere bereits als Emulsion vorliegen. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein.

Geeignete Silikone sind weiterhin aminofunktionelle Silikone (S4), insbesondere die Silikone, die unter der INCI-Bezeichnung Amodimethicone zusammengefasst sind. Darunter sind Silikone zu verstehen, welche mindestens eine, gegebenenfalls substituierte, Aminogruppe aufweisen.

Solche Silikone können z.B. durch die Formel (S4 - I)

M(RₐQ_{b}SiO_{(4-a-b)/2)})ₓ(R_{c}SiO_{(4-c)/2)})_{y}M (S4-I)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹Z ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silikon-Endgruppe ist, wie sie im Stand der Technik bekannt ist, vorzugsweise Trimethylsiloxy.

Z ist ein organischer, aminofunktioneller Rest, enthaltend mindestens eine funktionelle Aminogruppe. Eine mögliche Formel für Z ist NH(CH₂)_{z}NH₂, worin z für eine ganze Zahl von 1 bis 50 steht. Eine andere mögliche Formel für Z ist -NH(CH₂)_{z}NH(CH ₂)_{zz}, worin sowohl z als auch zz unabhängig voneinander für eine ganze Zahl von 1 bis 50 stehen, wobei diese Struktur Diamino-Ringstrukturen umfasst, wie Piperazinyl. Z ist insbesondere bevorzugt ein -NHCH₂CH₂NH₂-Rest. Eine andere mögliche Formel für Z ist - N(CH₂)_{z}NX¹X² oder -NX¹X², worin X¹ und X² jeweils unabhängig voneinander ausgewählt ist aus Wasserstoff und einem Kohlenwasserstoffrest mit 1 bis etwa 6 Kohlenstoffatomen.

Ganz besonders bevorzugt steht Q für einen polaren, aminfunktionellen Rest der Formel - CH₂CH₂CH₂NHCH₂CH₂NH₂.

Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und besonders bevorzugt von etwa 1 : 15 bis etwa 1 : 20. Werden ein oder mehrere Silikone der obigen Formel eingesetzt, dann können die verschiedenen variablen Substituenten in der obigen Formel bei den verschiedenen Silikonkomponenten, die in der Silikonmischung vorhanden sind, verschieden sein.

Besonders bevorzugte aminofunktionelle Silikone entsprechen der Formel (S4 - III) 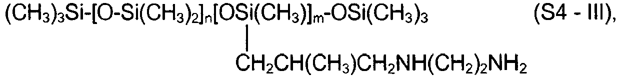 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet.

Besonders bevorzugt sind weiterhin aminofunktionelle Silikone der Formel (S4 - IV) 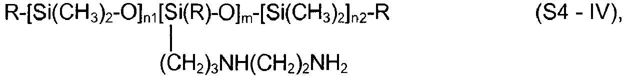 worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone bezeichnet und sind beispielsweise in Form einer Emulsion als Handelsprodukt Dow Corning^{®} 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Weitere geeignete Silikone sind beispielsweise
- oligomere Polydimethylcyclosiloxane (INCI-Bezeichnung: Cyclomethicone), insbesondere die tetramere und die pentamere Verbindung, die als Handelsprodukte DC 245 Fluid, DC 344 bzw. DC 345 von Dow Corning vertrieben werden,
- Hexamethyl-Disiloxan (INCI-Bezeichnung: Hexamethyldisiloxane), z. B. das unter der Bezeichnung Abil^{®} K 520 vertriebenen Produkt,
- Polyphenylmethylsiloxane (INCl-Bezeichnung: Phenyl Trimethicone), z. B. das Handelsprodukt DC 556 Cosmetic Grade Fluid von Dow Corning,
- Ester sowie Partialester der Silikon-Glykol-Copolymere, wie sie beispielsweise von der Firma Fanning unter der Handelsbezeichnung Fancorsil^{®} LIM (INCI-Bezeichnung: Dimethicone Copolyol Meadowfoamate) vertrieben werden,
- anionische Silikonöle, wie beispielsweise das Produkt Dow Corning^{®}1784.

Gemäß einer bevorzugten Ausführungsform enthält das erfindungsgemäße Mittel mindestens zwei unterschiedliche Silikonderivate, insbesondere bevorzugt eine Kombination aus einem flüchtigen und einem nichtflüchtigen Silikon. Flüchtig im Sinne der Erfindung sind solche Silikone, die eine Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning^{®}1401, Dow Corning^{®}1403 und Dow Corning^{®}1501, jeweils Mischungen aus einem Cyclomethicone und einem Dimethiconol) erhältlich.

Bevorzugte Mischungen verschiedener Silikone sind beispielsweise Dimethicone und Dimethiconole, lineare Dimethicone und cyclische Dimethiconole. Eine ganz besonders bevorzugte Mischung von Silikonen besteht aus mindestens einem cyclischen Dimethiconol und/oder Dimethicon, mindestens einem weiteren nicht cyclischen Dimethicon und/oder Dimethiconol sowie mindestens einem aminofunktionellen Silikon.

Werden unterschiedliche Silikone als Mischung verwendet, so ist das Mischungsverhältnis weitgehend variabel. Bevorzugt werden jedoch alle zur Mischung verwendeten Silikone in einem Verhältnis von 5 : 1 bis 1 : 5 im Falle einer binären Mischung eingesetzt. Ein Verhältnis von 3 : 1 bis 1 : 3 ist besonders bevorzugt. Ganz besonders bevorzugte Mischungen enthalten alle in der Mischung enthaltenen Silikone weitestgehend in einem Verhältnis von etwa 1 : 1, jeweils bezogen auf die eingesetzten Mengen in Gew.-%.

Die Mittel enthalten die Silikone bevorzugt in Mengen von 1 bis 25 Gew.-%, besonders bevorzugt von 5 bis 20 Gew.-% und insbesondere bevorzugt von 7 bis 15 Gew.-%, bezogen auf das gesamte Mittel.

Obwohl das erfindungsgemäße Mittel als Pflegestoff vorzugsweise ein Silikonderivat enthält, ist es auch möglich, dass das Mittel statt oder neben einer Silikonkomponente mindestens einen Pflegestoff einer anderen Verbindungsklasse enthält.

Als Pflegestoff einer anderen Verbindungsklasse kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Proteinhydrolysate pflanzlichen Ursprungs, z. B. Soja-, Mandel-, Erbsen-, Kartoffel- und Weizenproteinhydrolysate, sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff einer anderen Verbindungsklasse sind weiterhin kationische Tenside geeignet. Neben den Silikonölen und/oder Silikongummis sind die kationischen Tenside eine bevorzugte Klasse an Pflegestoffen.

Erfindungsgemäß bevorzugt sind kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Als Pflegestoff eignen sich ebenfalls pflegende Polymere. Es sei an dieser Stelle darauf hingewiesen, dass einige pflegende Polymere auch filmbildende und/oder festigende Eigenschaften aufweisen, und daher auch als filmbildende und/oder festigende Polymere eingesetzt werden können.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 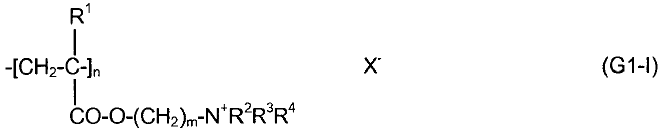
in der R¹= -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliche Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silikon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugt eingesetzte kationische Polymere sind kationische Cellulose-Derivate und Chitosan und dessen Derivate, insbesondere die Handelsprodukte Polymer^{®} JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein.
Ganz besonders bevorzugt sind die kationischen Proteinhydrolysate und -derivate auf pflanzlicher Basis.

Bevorzugt eingesetzte amphotere Polymere sind solche Polymerisate, die sich im wesentlichen zusammensetzen aus
(a) Monomeren mit quartären Ammoniumgruppen der allgemeinen Formel (II),

   R¹-CH=CR²-CO-Z-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ jeweils unabhängig voneinander für eine Alkylgruppe mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (III),

   R⁶-CH=CR⁷-COOH (III)

   in denen R⁶ und R⁷ unabhängig voneinander für Wasserstoff oder eine Methylgruppe stehen.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyltrimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Mittel enthalten die pflegenden, kationischen und/oder amphoteren Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden. Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt.

Ganz besonders bevorzugt wird als Pflegestoff D-Panthenol, gegebenenfalls in Kombination mit mindestens einem der oben genannten Silikonderivate eingesetzt.

Als Pflegestoff können die erfindungsgemäßen Mittel weiterhin mindestens einen Pflanzenextrakt enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt. Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders geeignet sind die Extrakte aus Grünem Tee, Mandel, Aloe Vera, Kokosnuss, Mango, Aprikose, Limone, Weizen, Kiwi und Melone.
Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.
Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.
Weiterhin kann es bevorzugt sein, in den erfindungsgemäßen Mitteln Mischungen aus mehreren, insbesondere aus zwei, verschiedenen Pflanzenextrakten einzusetzen.

Als Pflegestoff eignen sich weiterhin Ectoin oder Ectoinderivate, Allantoin, Taurin und/oder Bisabolol.

Die erfindungsgemäßen Mittel enthalten diese Pflegestoffe bevorzugt in Mengen von 0,001 bis 2, insbesondere von 0,01 bis 0,5 Gew.-%, jeweils bezogen auf die gesamte Anwendungszubereitung.

Auch Mono- bzw. Oligosaccharide können als Pflegestoff in den erfindungsgemäßen Mitteln eingesetzt werden. Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten. Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist. Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside. Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).
Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten. Erfindungsgemäß geeignete Lipide sind Phospholipide, beispielsweise Sojalecithin, Ei-Lecithin und Kephäline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben.
Die erfindungsgemäßen Mittel enthalten die Lipide bevorzugt in Mengen von 0,01 - 10 Gew.-%, insbesondere 0,1 - 5 Gew.-%, bezogen auf die gesamte Anwendungszubereitung.

Weiterhin sind als Pflegestoff Ölkörper geeignet. Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ -. Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 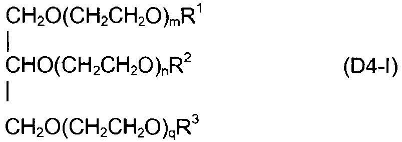 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.
Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Das Mittel kann überdies ein Enzym als Pflegestoff enthalten. Erfindungsgemäß besonders bevorzugte Enzyme sind ausgewählt aus einer Gruppe, die gebildet wird aus Proteasen, Lipasen, Transglutaminase, Oxidasen und Peroxidasen.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelte Haut oder die Haare vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern. Bevorzugt sind solche UV-Filter, deren molarer Extinktionskoeffizient am Absorptionsmaximum oberhalb von 15 000, insbesondere oberhalb von 20 000, liegt.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

Die erfindungsgemäßen Mittel können weiterhin mindestens ein Tensid enthalten.

Beispielsweise können kationische Tenside eingesetzt werden, wie sie bereits oben als geeignete Pflegestoffe beschrieben sind. Bezüglich der bevorzugten kationischen Tenside und der eingesetzten Mengen gelten obige Ausführungen entsprechend.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Ätomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 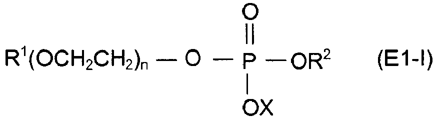 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Monoglyceridsulfate und Monoglyceridethersulfate der Formel (E1-III) 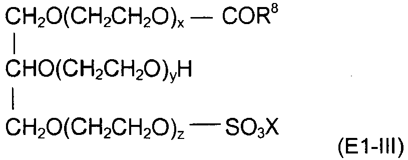 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈- C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Bevorzugt sind Alkyloligoglucoside auf Basis von gehärtetem C_{12/14}-Kokosalkohol mit einem DP von 1 bis 3.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung, enthalten. Mengen von 0,5 bis 15 Gew.-% sind besonders bevorzugt, und ganz besonders bevorzugt sind Mengen von 0,5 bis 7,5 Gew.-%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, eingesetzt. Dabei hängt die eingesetzte Menge wesentlich davon ab, welchen Zweck das erfindungsgemäße Mittel erfüllt. Handelt es sich um ein Shampoo oder ein anderes reinigendes Mittel, sind auch Tensidmengen über 45 Gew.-% üblich.

Die Mittel können weiterhin mindestens einen Emulgator enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 100 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- lineare und verzweigte Fettsäuren mit 8 bis 30 C - Atomen und deren Na-, K-, Ammonium-, Ca-, Mg- und Zn - Salze.

Die Emulgatoren werden bevorzugt in Mengen von 0,1 bis 25 Gew.-%, insbesondere 0,1 bis 3 Gew.-%, bezogen auf die jeweilige gesamte Zusammensetzung, eingesetzt.

Bevorzugt sind nichtionogene Emulgatoren mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen. Nichtionogene Emulgatoren mit einem HLB-Wert von 10 bis 16 sind erfindungsgemäß besonders bevorzugt.

Es kann weiterhin erforderlich sein, dass die erfindungsgemäßen Mittel weiterhin mindestens ein Verdickungsmittel enthalten.

Geeignete Verdickungsmittel sind beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate. Auch die Polymere, die als mögliche Pflegestoffe beschrieben wurden oder im Folgenden als filmbildende und/oder festigende Polymere aufgezählt werden, können verdickende Eigenschaften aufweisen, und können gegebenenfalls als Verdickungsmittel Verwendung finden.

Auch die Zugabe filmbildender und/oder festigender Polymere ist möglich.

Als filmbildende und/oder festigende Polymere können alle in dieser Funktion bekannten Polymere eingesetzt werden, wobei diese sowohl permanent als auch temporär kationisch, anionisch, nichtionisch oder amphoter sein können. Bei der Verwendung von mindestens zwei filmbildenden und/oder festigenden Polymeren können diese selbstverständlich unterschiedliche Ladungen aufweisen. Erfindungsgemäß bevorzugt kann es sein, wenn ein ionisches filmbildendes und/oder festigendes Polymer mit einem amphoteren und/oder nichtionischem filmbildenden und/oder festigenden Polymer gemeinsam verwendet wird. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Da Polymere häufig multifunktional sind, können deren Funktionen nicht immer klar und eindeutig voneinander abgegrenzt werden. Insbesondere gilt dies für filmbildende und festigende Polymere. Dennoch sollen beispielhaft einige filmbildende Polymere beschrieben werden. Es wird an dieser Stelle jedoch explizit darauf verwiesen, dass im Rahmen der vorliegenden Erfindung sowohl filmbildende als auch festigende Polymere wesentlich sind. Da beide Eigenschaften auch nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Alkohol oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nicht-ionisch, permanent kationisch oder temporär kationisch geladen sein.

Geeignete synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C₁-bis C₇-Alkylgruppen, besonders bevorzugt C₁- bis C₃-Alkylgruppen sind.

Beispielhaft seien genannt Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine^{®} P 191 von der Firma CHEM-Y, Emmerich, oder Sepigel^{®} 305 von der Firma Seppic vertrieben werden; Polyvinylalkohole, die beispielsweise unter den Handelsbezeichnungen Elvanol^{®} von Du Pont oder Vinol^{®} 523/540 von der Firma Air Products vertrieben werden sowie Polyethylenglykol/Polypropylenglykol-Copolymere, die beispielsweise, unter den Handelsbezeichnungen Ucon^{®} der Union Carbide vertrieben werden.

Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird.

Festigende Polymere tragen zum Halt und/oder zum Aufbau des Haarvolumens und der Haarfülle der Gesamtfrisur bei. Diese so genannten festigenden Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Substanzen, welche dem Haar weiterhin hydrophobe Eigenschaften verleihen, sind hierbei bevorzugt, weil sie die Tendenz des Haares, Feuchtigkeit, also Wasser, zu absorbieren, verringern. Dadurch wird das schlaffe Herunterhängen der Haarsträhnen vermindert und somit ein lang anhaltender Frisurenaufbau und -erhalt gewährleistet. Als Testmethode hierfür wird häufig der so genannte curI-retention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch. Wegen der Bedeutung gerade der festigenden Polymere sollen diese daher explizit in Form ihrer INCI - Namen aufgelistet werden. In dieser Liste der erfindungsgemäß bevorzugt zu verwendenden Polymere finden sich somit selbstverständlich gerade auch die genannten filmbildenden Polymere wieder.

Bevorzugte filmbildende und/oder festigende Polymere sind die Polyvinylpyrrolidone, Vinylpyrrolidon-Vinylacetat-Copolymere, Vinylacetat-Crotonsäure-Copolymere, Vinylcaprolactam-Vinylpyrrolidon-Dimethylaminoethylmethacrylat-Copolymere, Octylacrylamid-Acrylat-Butylaminoethyl-Methacrylat-Copolymere und quaternierte Vinylpyrrolidon-Dimethylaminoethylmethacrylat-Copolymere.

Besonders bevorzugt handelt es sich bei dem filmbildenden und/oder festigenden Polymer um die Vinylpyrrolidon-Vinylacetat-Copolymeren Luviskol^{®} VA 37 oder PVP/VA Copolymer 60/40 W NP, das Vinylacetat-Crotonsäure-Copolymer, das unter der Handelsbezeichnung Aristoflex^{®} A 60 vertrieben wird, das Vinylcaprolactam-Vinylpyrrolidon-Dimethylaminoethylmethacrylat-Copolymer mit der Handelsbezeichnung Advantage^{®} LC-E, das unter der Bezeichnung Amphomer^{®} erhältliche amphotere Octylacrylamid-Acrylat-Butylaminoethyl-Methacrylat-Copolymer oder das durch Umsetzung mit Diethylsulfat quaternierte Vinylpyrrolidon-Dimethylaminoethylmethacrylat-Copolymer, das unter der Handelsbezeichnung Gafquat^{®} 755N vertrieben wird.

Insbesondere bevorzugt handelt es sich bei dem filmbildenden und/oder festigenden Polymer um ein Vinylpyrrolidon-Vinylacetat-Copolymer.

Die Mittel können neben den genannten Komponenten weiterhin alle für entsprechende kosmetische Mittel bekannten Wirk-, Zusatz- und Hilfsstoffe enthalten. Dies sind beispielsweise
- Strukturanten wie Maleinsäure und Milchsäure,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und Basen,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wasserstoffperoxid und andere Oxidationsmittel,
- Treibmittel wie Propan-Butan-Gemische, N₂O, Dimethylether, CO₂ und Luft.

Bezüglich weiterer fakultativer Komponenten sowie der eingesetzten Mengen dieser Komponenten wird ausdrücklich auf die dem Fachmann bekannten einschlägigen Handbücher verwiesen.

Es ist selbstverständlich, dass in Bezug auf die optionalen Bestandteile der erfindungsgemäßen Mittel darauf zu achten ist, dass es nicht zu unerwünschten Wechselwirkungen mit den zwingenden Bestandteilen, insbesondere dem Wasserstoffperoxid kommt.

Die Formulierung der erfindungsgemäßen Mittel kann in allen für kosmetische Mittel üblichen Formen erfolgen, beispielsweise in Form von Lösungen, die als Haarwasser oder Pump- oder Aerosolspray auf das Haar aufgebracht werden können, in Form von Emulsionen, Wachsen, Gelen oder auch tensidhaltigen schäumenden Lösungen oder anderen Zubereitungen, die für die Anwendung auf dem Haar geeignet sind.

In einer bevorzugten Ausführungsform handelt es sich bei den erfindungsgemäßen Mitteln um Mittel zur Durchführung des oxidativen Schrittes im Rahmen einer dauerhaften Verformung keratinischer Fasern, d.h. um sogenannte Fixiermittel.

Ein zweiter Gegenstand der Erfindung ist demnach die Verwendung der erfindungsgemäßen Mittel zur Durchführung des oxidativen Schrittes im Rahmen einer dauerhaften Verformung keratinischer Fasern.

Ein weiterer Gegenstand der vorliegenden Erfindung ist ein kosmetisches Verfahren zum dauerhaften Verformen keratinhaltiger Fasern, insbesondere menschlicher Haare, wobei
(i) vor, während und/oder nach einer mechanischen Verformung der keratinhaltigen Fasern eine wässrige, keratinreduzierende Zusammensetzung, enthaltend mindestens eine keratinreduzierende Verbindung auf die Fasern aufgetragen wird,
(ii) die Fasern nach einer Einwirkzeit Z1 gespült und gegebenenfalls getrocknet werden, und
(iii) schließlich eine oxidierende Zusammensetzung, enthaltend mindestens eine oxidierende Verbindung, auf die Fasern aufgetragen und nach einer Einwirkzeit Z2 wieder abgespült wird,
wobei es sich bei der oxidierenden Zusammensetzung um ein erfindungsgemäßes Mittel handelt.

Die mechanische Verformung erfolgt vorzugsweise unter Zuhilfenahme von Verformungshilfsmitteln.

Verformungshilfsmittel im Sinne des erfindungsgemäßen Verfahrens können z.B. Lockenwickler oder Papilloten im Falle einer Dauerwelle, oder Hilfsmittel für eine mechanische Glättung, wie ein Kamm oder eine Bürste, ein Glättungsboard oder ein beheizbares Glättungseisen im Falle einer Haarglättung sein.

Wenn die Verformungshilfsmittel, beispielsweise Wickler, im Rahmen eines Dauerwellverfahrens für einen längeren Zeitraum an der Faser befestigt werden, so kann es zweckmäßig sein, diese Verformungshilfsmittel vor, während oder nach dem Aufbringen der oxidierenden Zusammensetzung zu entfernen. Es kann in diesem Zusammenhang vorteilhaft sein, die Verformungshilfsmittel während des Einwirkens der oxidativen Zusammensetzung im Haar zu belassen, sie danach zu entfernen und danach den Oxidationsschritt als sogenannten Nachfixierschritt zu wiederholen.

In Schritt (i) des erfindungsgemäßen Verfahrens werden die keratinhaltigen Fasern mit einer wässrigen, keratinreduzierenden Zusammensetzung behandelt. Eine wässrige Zusammensetzung im Sinne der Erfindung enthält mindestens 50 Gew.% Wasser, bezogen auf das Gewicht der gesamten Zusammensetzung.

Die wässrige, keratinreduzierende Zusammensetzung enthält mindestens eine keratinreduzierende Verbindung. Die keratinreduzierenden Verbindungen werden dabei bevorzugt ausgewählt aus Verbindungen mit mindestens einer Thiolgruppe sowie deren Derivate, aus Sulfiten, Hydrogensulfiten und Disulfiten.

Verbindungen mit mindestens einer Thiolgruppe sowie deren Derivate sind beispielsweise Thioglykolsäure, Thiomilchsäure, Thioäpfelsäure, Phenylthioglykolsäure, Mercaptoethansulfonsäure sowie deren Salze und Ester (wie z.B. Isooctylthioglycolat und Isopropylthioglycolat), Cysteamin, Cystein, Bunte Salze und Salze der schwefligen Säure. Bevorzugt geeignet sind die Monoethanolammoniumsalze oder Ammoniumsalze der Thioglykolsäure und/oder der Thiomilchsäure sowie die freien Säuren. Diese werden in der wässrigen Zusammensetzung bevorzugt in Konzentrationen von 0,5 bis 2,0 mol/kg bei einem pH-Wert von 5 bis 12, insbesondere von 7 bis 9,5, eingesetzt. Zur Einstellung dieses pH-Wertes enthalten die wässrigen Zusammensetzungen üblicherweise Alkalisierungsmittel wie Ammoniak, Alkali- und Ammoniumcarbonate und -hydrogencarbonate oder organische Amine wie Monoethanolamin.

Beispiele für keratinreduzierende Verbindungen der Disulfite, die in der wässrigen Zusammensetzung enthalten sein können, sind Alkalidisulfite, wie z.B. Natriumdisulfit (Na₂S₂O₅) und Kaliumdisulfit (K₂S₂O₅), sowie Magnesiumdisulfit und Ammoniumdisulfit ((NH₄)₂S₂O₅). Ammoniumdisulfit kann dabei erfindungsgemäß bevorzugt sein. Beispiele für keratinreduzierende Verbindungen der Hydrogensulfite, die in der wässrigen Zusammensetzung enthalten sein können, sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit kann dabei ein besonders bevorzugtes Hydrogensulfit sein. Beispiele für keratinreduzierende Verbindungen der Sulfite, die in der wässrigen Zusammensetzung enthalten sein können, sind Sulfite als Alkali-, Ammonium- oder Alkanolammonium-Salz auf Basis eines C₂-C₄-Mono-, Di- oder Trialkanolamins. Ammoniumsulfit ist dabei bevorzugt. Der pH-Wert der wässrigen Zusammensetzung wird bei Verwendung von Sulfit und/oder Disulfit und/oder Hydrogensulft bevorzugt auf einen Wert im Neutralbereich von pH 5 bis 8, bevorzugt von pH 6 bis 7,5 eingestellt.

Bevorzugte C₂-C₄-Alkanolamine sind erfindungsgemäß 2-Aminoethanol (Monoethanolamin) und N,N,N-Tris(2-hydroxyethyl)amin (Triethanolamin). Monoethanolamin ist ein besonders bevorzugtes C₂-C₄-Alkanolamin, das insbesondere in einer Menge von 0,2 bis 6 Gew.-% bezogen auf die gesamte wässrige Zusammensetzung, eingesetzt wird.

Die in der wässrigen Zusammensetzung enthaltenen keratinreduzierenden Verbindungen werden besonders bevorzugt ausgewählt aus Thioglykolsäure, Thiomilchsäure und Cystein sowie deren Salze.

Die keratinreduzierende Verbindung wird bevorzugt in einer Menge von 1 bis 25 Gew.-%, besonders bevorzugt in einer Menge von 5 bis 15 Gew.-%, bezogen auf die gesamte wässrige, keratinreduzierende Zusammensetzung, eingesetzt.

Darüberhinaus kann die wässrige, keratinreduzierende Zusammensetzung weitere Komponenten enthalten, die die Wirkung der keratinreduzierenden Verbindung auf das Keratin fördern. Solche Komponenten sind z.B. Quellmittel für keratinhaltige Fasern wie z.B. C₁-C₆-Alkohole und wasserlösliche Glykole oder Polyole wie z.B. Glycerin, 1,2-Propylenglykol oder Sorbit und Harnstoff oder Harnstoffderivate wie z.B. Allantoin und Guanidin sowie Imidazol und dessen Derivate. In einer bevorzugten Ausführung enthält die wässrige Zusammensetzung 0,05 bis 5 Gew.-% 1,2-Propylenglykol und/oder 0,05 bis 5 Gew.-% Harnstoff. Die Mengenangaben beziehen sich jeweils auf die gesamte wässrige Zusammensetzung.

Die wässrige, keratinreduzierende Zusammensetzung kann weiterhin die bekannten Wirk-, Hilfs- und Zusatzstoffe enthalten, die üblicherweise Well- oder Glättungsmitteln zugegeben werden. Diesbezüglich wird auf obige Ausführungen zu den optionalen Inhaltsstoffen der erfindungsgemäßen Mittel verwiesen.

Die Einwirkzeit Z1 beträgt bevorzugt 5-60 Minuten, besonders bevorzugt 10-30 Minuten. Die Einwirkzeit Z2 beträgt bevorzugt 1-30 Minuten, besonders bevorzugt 1-15 Minuten.

Eine trockene keratinhaltige Faser liegt im Sinne der Erfindung dann vor, wenn die den Haaren anhaftenden Wasserreste soweit verdampft sind, dass die Haare einzeln fallen. Bevorzugt ist bei einer trockenen keratinhaltigen Faser entweder der Feuchtigkeitsgehalt der Faser mit der Feuchtigkeit der Luft im Wesentlichen im Gleichgewicht oder die Faser nimmt Feuchtigkeit aus der Luft der Umgebung auf.

In einer bevorzugten Ausführungsform des erfindungsgemäßen Verfahrens werden die keratinhaltigen Fasern vor der Durchführung von Schritt (i) des Verfahrens angefeuchtet. Dies kann durch Besprühen der Fasern mit einer Flüssigkeit, bevorzugt mit Wasser, geschehen.

Bevorzugterweise werden die Fasern mit einem herkömmlichen Shampoo shampooniert, gespült und dann mit einem Handtuch frottiert. Nach Abschluß des Frottierschritts bleibt eine fühlbare Restfeuchtigkeit im Haar zurück.

In einer weiteren bevorzugten Ausführungsform der Erfindung werden die keratinischen Fasern einer Temperaturbehandlung unterzogen. Als besonders vorteilhaft hat es sich erwiesen, die Temperaturbehandlung während der Einwirkung der wässrigen, keratinreduzierenden Zusammensetzung oder nach dem Ausspülen der wässrigen, keratinreduzierenden Zusammensetzung durchzuführen.

Die Temperaturbehandlung kann beispielsweise mittels heizbarer Wickler, der Zuführung erwärmter Luft, beispielsweise mittels Föhn oder Trockenhaube oder für den Fall, dass die keratinischen Fasern geglättet werden sollen, auch mit Hilfe entsprechend temperierter Platten, insbesondere Metall- oder Keramikplatten, erfolgen.

Bei der Temperaturbehandlung werden die keratinischen Fasern vorzugsweise auf eine Temperatur von 30°C bis 220°C erwärmt. Der bevorzugte Temperaturbereich hängt insbesondere davon ab, ob es sich bei der Verformung um eine Wellung oder eine Glättung handelt, und ob die Temperaturbehandlung während der Einwirkung der wässrigen, keratinreduzierenden Zusammensetzung oder nach dem Ausspülen der wässrigen, keratinreduzierenden Zusammensetzung durchgeführt wird.

Handelt es sich bei der Verformung um eine Wellung und wird die Temperaturbehandlung während der Einwirkung der wässrigen, keratinreduzierenden Zusammensetzung durchgeführt, ist ein Temperaturbereich von 30°C bis 80°C, insbesondere von 35°C bis 60°C, bevorzugt.
Handelt es sich bei der Verformung um eine Wellung und wird die Temperaturbehandlung nach dem Ausspülen der wässrigen, keratinreduzierenden Zusammensetzung durchgeführt, ist ein Temperaturbereich von 80°C bis 150°C, insbesondere von 80°C bis 140°C, bevorzugt.
Handelt es sich bei der Verformung um eine Glättung und wird die Temperaturbehandlung während der Einwirkung der wässrigen, keratinreduzierenden Zusammensetzung durchgeführt, sind Temperaturen von 30°C bis 80°C, insbesondere 35°C bis 60°C, bevorzugt.
Handelt es sich bei der Verformung um eine Glättung und wird die Temperaturbehandlung nach dem Ausspülen der wässrigen, keratinreduzierenden Zusammensetzung durchgeführt, sind Temperaturen von 120°C bis 220°C, insbesondere 130°C bis 200°C, bevorzugt.

Im Anschluss an die Durchführung der erfindungsgemäßen Verfahren können die keratinischen Fasern in üblicher Weise nachbehandelt werden. Beispielsweise kann die Anwendung eines handelsüblichen Conditioner vorteilhaft sein. Eine Behandlung mit einem Conditioner kann auch als Zwischenbehandlung erfolgen.

Die nachfolgenden Beispiele sollen den Gegenstand der vorliegenden Erfindung weiter erläutern ohne ihn in irgendeiner Weise zu beschränken.

### Beispiele

### 1. Wirknachweise

### 1.1 Zug-Dehnungs-Messung

Etwa 30 cm lange Haarsträhnen wurden einer dreifachen Dauerwellbehandlung unterzogen. Dabei kam Haar zum Einsatz, das unter der Bezeichnung "european natural dark brown hair", Typ 6634 vom Anbieter Alkinco kommerziell erhältlich ist.

Das Haar wurde zunächst 30 Minuten bei 32°C mit der unter der Bezeichnung Professionelle Perm Lotion N von der Firma Henkel & Schwarzkopf kommerziell erhältlichen Dauerwelllösung behandelt, dann 5 Minuten mit Leitungswasser einer Temperatur von 38°C gespült, mit einem Handtuch getrocknet, anschließend für 10 Minuten mit Fixierlösung behandelt und schließlich wieder 5 Minuten mit Leitungswasser einer Temperatur von 38°C gespült. Nach Lagerung der Haare für 24 Stunden bei Raumtemperatur wurde die gesamte Behandlung inklusive Lagerung zweimal wiederholt.

Die verwendeten Fixierlösungen (V1 und V2: Vergleichszubereitungen; E1: erfindungsgemäßes Fixiermittel) sind in folgender Tabelle wiedergegeben:

| **Rohstoffe** | **V1** [Gew..-%] | **V2** [Gew..-%] | **E1** [Gew..-%] |
|---|---|---|---|
| Texapon NSO¹ | 3,0 | 3,0 | 3,0 |
| 1-Hydroxyethan-1,1-diphosphonsäure² | 0,8 | 0,8 | 0,8 |
| Dipicolinsäure | 0,05 | 0,05 | 0,05 |
| NaOH³ | 0,42 | 0,42 | 0,42 |
| Koffein | - | 1,0 | 1,0 |
| H₂O₂⁴ | 5,0 | - | 0,5 |
| H₂O | Ad 100 | Ad 100 | Ad 100 |

| | | | |
|---|---|---|---|
| ¹ Laurylethersulfat, Natriumsalz (ca. 27,5% Aktivsubstanz; INCI-Bezeichnung: Sodium Laureth Sulfate) (Cognis) ² 60-%ige wässrige Lösung ³ 45-%ige wässrige Lösung ⁴ 50-%ige wässrige Lösung | | | |

Zur Bestimmung der Haarschädigung wurden jeweils 40 nasse Einzelhaare jeweils vor und nach dem oben beschriebenen 3-fachen Dauerwellprozess unter Verwendung der Fixierlösungen V1, V2 bzw. E1 einer Zug-Dehnungs-Messung mittels des kommerziell erhältlichen Messgeräts "MTT 675 automated miniature tensile tester" der Firma DiaStron unterzogen, der Haarquerschnitt der unbehandelten und der dauergewellten Haare vermessen und das E-Modul ermittelt. Die Zug-Dehnungs-Messung erfolgt dabei zerstörungsfrei bis zu einer Dehnung von 1 %.

Die Mittelwerte der 40 Einzelmessungen der ermittelten E-Module sind in folgender Tabelle wiedergegeben. Die statistische Betrachtung mittels t-Test ergab für alle Messreihen, dass sie mit einer Wahrscheinlichkeit >99.99% voneinander unterschieden sind. Somit weisen alle Messreihen statistisch signifikante Unterschiede voneinander auf:

| **Fixiermittel** | **V1** | **V2** | **E1** |
|---|---|---|---|
| **E-Modul [N/m²]** vor Dauerwellung | 21,4 x 10⁸ | 21,7 x 10⁸ | 21,5 x 10⁸ |
| **E-Modul [N/m²]** nach Dauerwellung | 4,04 x 10⁸ | 7,51 x 10⁸ | 9,60 x 10⁸ |
| **Änderung E-Modul** [%] | -81 | -65 | -55 |

Es zeigt sich, dass bei Verwendung des erfindungsgemäßen Fixiermittels E1, enthaltend 1 Gew.- % Koffein und 0,25 Gew.-% Wasserstoffperoxid das E-Modul im Vergleich zum Einsatz eines üblichen Fixiermittels V1 enthaltend 2,5 Gew.-% Wasserstoffperoxid in Bezug auf das E-Modul eines unbehandelten Haars deutlich weniger abfällt. Das Haar wird demnach weniger geschädigt. Überraschenderweise gilt dies auch im Vergleich zum Einsatz eines Fixiermittels V2, enthaltend 1 Gew.-% Koffein als Oxidationsmittel. Dass trotz Zugabe von Wasserstoffperoxid die Schädigung des Haars geringer ausfällen würde, war nicht zu erwarten.

### 1.2 Wellwirkung

Etwa 30 cm lange Haarsträhnen wurden der folgenden Dauerwellbehandlung unterzogen. Dabei kam Haar zum Einsatz, das unter der Bezeichnung "european natural dark brown hair", Typ 6634 vom Anbieter Alkinco kommerziell erhältlich ist.

Das Haar wurde zunächst 30 Minuten bei 32°C mit der unter der Bezeichnung Professionelle Perm Lotion N von der Firma Henkel & Schwarzkopf kommerziell erhältlichen Dauerwelllösung behandelt, auf Spiralwickler gewickelt, dann nach 30 Minuten Einwirkzeit bei 32°C mit Leitungswasser einer Temperatur von 38°C gespült, mit einem Handtuch getrocknet, anschließend für 10 Minuten mit einer Fixierlösung (*vide infra*) behandelt und schließlich wieder 5 Minuten mit Leitungswasser einer Temperatur von 38°C gespült. Nach Lagerung der Haare für 24 Stunden bei Raumtemperatur wurde die Wellhaltbarkeit geprüft.

Die verwendeten Fixierlösungen (V1 Vergleichszubereitung; E2, E3, E4: erfindungsgemäße Fixiermittel) sind in folgender Tabelle wiedergegeben:

| **Rohstoffe** | **V1** [Gew..-%] | **E2** [Gew..-%] | **E3** [Gew..-%] | **E4** [Gew..-%] |
|---|---|---|---|---|
| Texapon NSO¹ | 3,0 | 3,0 | 3,0 | 3,0 |
| 1-Hydroxyethan-1,1-diphosphonsäure² | 0,8 | 0,8 | 0,8 | 0,8 |
| Dipicolinsäure | 0,05 | 0,05 | 0,05 | 0,05 |
| NaOH³ | 0,42 | 0,42 | 0,42 | 0,42 |
| Koffein | - | 1,0 | 1,0 | 1,0 |
| H₂O₂⁴ | 5,0 | 0,05 | 0,2 | 2,0 |
| H₂O | Ad 100 | Ad 100 | Ad 100 | Ad 100 |

| | | | | |
|---|---|---|---|---|
| ¹ Laurylethersulfat, Natriumsalz (ca. 27,5% Aktivsubstanz; INCI-Bezeichnung: Sodium Laureth Sulfate) (Cognis) ² 60-%ige wässrige Lösung ³ 45-%ige wässrige Lösung ⁴ 50-%ige wässrige Lösung | | | | |

Die 4 Teststrähnen wurden bei 90 % relativer Luftfeuchtigkeit und 35°C gelagert. Nach einer Stunde Lagerung wiesen alle Strähnen eine visuell identische Wellwirkung auf.

Durch Einsatz der erfindungsgemäßen Fixiermittel konnte bei reduzierter Menge Wasserstoffperoxid unter zusätzlichem Einsatz von Koffein die Effektivität und Haltbarkeit der Wellwirkung im Vergleich zu einer herkömmlichen Dauerwellfixierung (V1) trotz des geringen Gehaltes an Oxidationsmittel beibehalten werden.

## Patentansprüche

1. Kosmetisches Mittel, enthaltend in einem kosmetisch akzeptablen Träger, jeweils bezogen auf das gesamte Mittel
a) 0,03 bis 10 Gew.-% Purin und/oder Purinderivat(e) und
b) Wasserstoffperoxid in einer Menge von 0,001 bis höchstens 5 Gew.-%.

2. Mittel nach Anspruch 1, **dadurch gekennzeichnet, dass** es bezogen auf das gesamte Mittel 0,5 bis 2,5 Gew.-% Purin(e) und/oder Purinderivat(e) enthält.

3. Mittel nach einem der Ansprüche 1 bis 2, **dadurch gekennzeichnet, dass** es Purin und/oder Purinderivat(e) der Formel (I) enthält 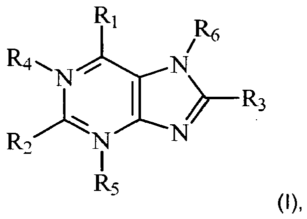 wobei die Reste R¹, R² und R³ unabhängig voneinander ausgewählt sind aus -H, - OH, - NH₂, -SH und die Reste R⁴, R⁵ und R⁶ unabhängig voneinander ausgewählt sind aus -H, - CH₃ und -CH₂-CH₃.

4. Mittel nach einem der Ansprüche 1 bis 3, **dadurch gekennzeichnet, dass** es sich bei dem Purin und/oder Purinderivat um Xanthin und/oder ein Xanthinderivat der Formel (I) handelt, wobei R¹ und R² für - OH und R³ für -H stehen.

5. Mittel nach einem der Ansprüche 3 bis 4, **dadurch gekennzeichnet, dass** in Formel (I) R⁴, R⁵ und R⁶ unabhängig voneinander ausgewählt sind aus -H und -CH₃.

6. Mittel nach einem der Ansprüche 1 bis 5, **dadurch gekennzeichnet, dass** es als Purin und/oder Purinderivat Koffein enthält.

7. Mittel nach einem der Ansprüche 1 bis 6, **dadurch gekennzeichnet, dass** es bezogen auf das gesamte Mittel 0,7 bis 4,0 Gew.-%, vorzugsweise 1,0 bis 3,0 Gew.-%, besonders bevorzugt 2,0 bis 3,0 Gew.-% Wasserstoffperoxid enthält.

8. Mittel nach einem der Ansprüche 1 bis 6, **dadurch gekennzeichnet, dass** es bezogen auf das gesamte Mittel 0,1 bis 0,4 Gew.-%, vorzugsweise 0,1 bis 0,35 Gew.-%, besonders bevorzugt 0,1 bis 0,3 Gew.-% Wasserstoffperoxid enthält.

9. Mittel nach einem der Ansprüche 1 bis 6, **dadurch gekennzeichnet, dass** es bezogen auf das gesamte Mittel 0,001 bis 0,1 Gew.-%, vorzugsweise 0,005 bis 0,075 Gew.-%, besonders bevorzugt 0,0075 bis 0,05 Gew.-% Wasserstoffperoxid enthält.

10. Mittel nach einem der Ansprüche 1 bis 9, **dadurch gekennzeichnet, dass** es sich um ein Mittel zur Durchführung des oxidativen Schrittes im Rahmen einer dauerhaften Verformung keratinhaltiger Fasern handelt.

11. Verwendung eines Mittels gemäß wenigstens eines der Ansprüche 1 bis 10 zur Durchführung des oxidativen Schrittes im Rahmen einer dauerhaften Verformung keratinhaltiger Fasern.

12. Verfahren zum dauerhaften Verformen keratinhaltiger Fasern, insbesondere menschlicher Haare, wobei
(i) vor, während und/oder nach einer mechanischen Verformung der keratinhaltigen Fasern eine wässrige, keratinreduzierende Zusammensetzung, enthaltend mindestens eine keratinreduzierende Verbindung auf die Fasern aufgetragen wird,
(ii) die Fasern nach einer Einwirkzeit Z1 gespült und gegebenenfalls getrocknet werden, und
(iii) schließlich eine oxidierende Zusammensetzung, enthaltend mindestens eine oxidierende Verbindung, auf die Fasern aufgetragen und nach einer Einwirkzeit Z2 wieder abgespült wird,
**dadurch gekennzeichnet, dass** es sich bei der oxidierenden Zusammensetzung um ein Mittel gemäß wenigstens eines der Ansprüche 1 bis 10 handelt.

## Claims

1. Cosmetic agent containing, in a cosmetically acceptable carrier, in each case based on the total agent:
a) 0.03 to 10 wt.% purine and/or purine derivative(s); and
b) hydrogen peroxide in an amount of 0.001 up to at most 5 wt.%.

2. Agent according to claim 1, **characterised in that**, based on the total agent, it contains 0.5 to 2.5 wt.% purine(s) and/or purine derivative(s).

3. Agent according to either claim 1 or claim 2, **characterised in that** it contains purine and/or purine derivative(s) of formula (I) 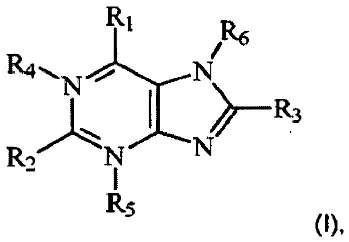 the residues R¹, R², and R³ being selected, irrespective of one another, from among -H, -OH, -NH₂ and -SH, and the residues R⁴, R⁵ and R⁶ being selected, irrespective of one another, from among -H, -CH₃ and CH₂-CH₃.

4. Agent according to any of claims 1 to 3, **characterised in that** the purine and/or purine derivative is xanthine and/or a xanthine derivative of formula (I), R¹ and R² standing for -OH and R³ for -H.

5. Agent according to any of claims 3 to 4, **characterised in that** in formula (I) R⁴, R⁵, and R⁶ are selected, irrespective of one another, from among -H and -CH₃.

6. Agent according to any of claims 1 to 5, **characterised in that** it contains caffeine as the purine and/or purine derivative.

7. Agent according to any of claims 1 to 6, **characterised in that**, based on the total agent, it contains 0.7 to 4.0 wt.%, preferably 1.0 to 3.0 wt.%, particularly preferably 2.0 to 3.0 wt.% hydrogen peroxide.

8. Agent according to any of claims 1 to 6, **characterised in that**, based on the total agent, it contains 0.1 to 0.4 wt.%, preferably 0.1 to 0.35 wt.%, particularly preferably 0.1 to 0.3 wt.% hydrogen peroxide.

9. Agent according to any of claims 1 to 6, **characterised in that**, based on the total agent, it contains 0.001 to 0.1 wt.%, preferably 0.005 to 0.075 wt.%, particularly preferably 0.0075 to 0.05 wt.% hydrogen peroxide.

10. Agent according to any of claims 1 to 9, **characterised in that** it is an agent for carrying out the oxidation step in the process of permanently shaping keratine-containing fibres.

11. Use of an agent according to at least one of claims 1 to 10 for carrying out the oxidation step in the process of permanently shaping keratine-fibres.

12. Method for permanently shaping keratine-containing fibres, in particular human hair, wherein:
(i) prior to, during and/or after mechanical shaping of the keratine-containing fibres, an aqueous, keratine-reducing composition containing at least one keratine-reducing compound is applied to the fibres;
(ii) the fibres are rinsed following elapse of an action time Z1 and optionally dried; and
(ii) finally an oxidising composition, containing at least one oxidising compound, is applied to the fibres and rinsed off again following elapse of an action time Z2;
**characterised in that** the oxidising compound is an agent according to at least one of claims 1 to 10.

## Revendications

1. Agent cosmétique, contenant dans un support cosmétiquement acceptable, à chaque fois par rapport à la totalité de l'agent
a) 0,03 à 10% en poids de purine et/ou de dérivé(s) de purine et
b) du peroxyde d'hydrogène en une quantité de 0,001 à maximum 5% en poids.

2. Agent selon la revendication 1, **caractérisé en ce qu'**il contient, par rapport à la totalité de l'agent, 0,5 à 2,5% en poids de purine et/ou de dérivé(s) de purine.

3. Agent selon l'une quelconque des revendications 1 à 2, **caractérisé en ce qu'**il contient de la purine et/ou un/des dérivé(s) de purine de formule (I) 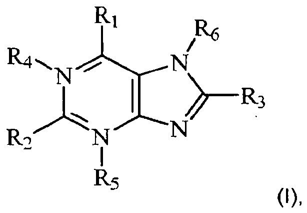 les résidus R¹, R² et R³ étant choisis, indépendamment les uns des autres, parmi -H, -OH, -NH₂, -SH et les résidus R⁴, R⁵ et R⁶ étant choisis, indépendamment les uns des autres, parmi -H, -CH₃ et -CH₂-CH₃.

4. Agent selon l'une quelconque des revendications 1 à 3, **caractérisé en ce qu'**il s'agit, pour la purine et/ou le dérivé de purine, de xanthine et/ou d'un dérivé de xanthine de formule (I), R¹ et R² représentant -OH et R³ représentant -H.

5. Agent selon l'une quelconque des revendications 3 à 4, **caractérisé en ce que**, dans la formule (I), R⁴, R⁵ et R⁶ sont choisis, indépendamment les uns des autres, parmi -H et -CH₃.

6. Agent selon l'une quelconque des revendications 1 à 5, **caractérisé en ce qu'**il contient, comme purine et/ou dérivé de purine, de la caféine.

7. Agent selon l'une quelconque des revendications 1 à 6, **caractérisé en ce qu'**il contient, par rapport à la totalité de l'agent, 0,7 à 4,0% en poids, de préférence 1,0 à 3,0% en poids, de manière particulièrement préférée 2,0 à 3,0% en poids de peroxyde d'hydrogène.

8. Agent selon l'une quelconque des revendications 1 à 6, **caractérisé en ce qu'**il contient, par rapport à la totalité de l'agent, 0,1 à 0,4% en poids, de préférence 0,1 à 0,35% en poids, de manière particulièrement préférée 0,1 à 0,3% en poids de peroxyde d'hydrogène.

9. Agent selon l'une quelconque des revendications 1 à 6, **caractérisé en ce qu'**il contient, par rapport à la totalité de l'agent, 0,001 à 0,1% en poids, de préférence 0,005 à 0,075% en poids, de manière particulièrement préférée 0,0075 à 0,05% en poids de peroxyde d'hydrogène.

10. Agent selon l'une quelconque des revendications 1 à 9, **caractérisé en ce qu'**il s'agit d'un agent pour réaliser l'étape d'oxydation dans le cadre d'une mise en forme permanente de fibres kératiniques.

11. Utilisation d'un agent selon au moins l'une quelconque des revendications 1 à 10 pour la réalisation de l'étape d'oxydation dans le cadre d'une mise en forme permanente de fibres kératiniques.

12. Procédé pour la mise en forme permanente de fibres kératiniques, en particulier de cheveux humains, dans lequel
(i) on applique, avant, pendant et/ou après une mise en forme mécanique des fibres kératiniques, une composition aqueuse, réduisant la kératine, contenant au moins un composé réduisant la kératine sur les fibres,
(ii) les fibres sont rincées après un temps d'action Z1 et le cas échéant séchées, et
(iii) enfin, une composition oxydante, contenant au moins un composé oxydant, est appliquée sur les fibres et à nouveau éliminée par rinçage après un temps d'action Z2,
**caractérisé en ce qu'**il s'agit, pour la composition oxydante, d'un agent selon au moins l'une quelconque des revendications 1 à 10.
